# EUROPEAN PATENT APPLICATION

(11) **EP 3 698 742 A1**
(43) Date of publication of application: **26.08.2020**
(21) Application number: 19157961.4
(22) Date of filing: 19.02.2019
(51) Int. Cl.: A61B 17/66, A61B 17/88

(54) **ALIGNMENT DEVICE FOR BONES**

(71) Applicant: Arthrex Inc, Naples, FL 34108-1945 (US)
(72) Inventor: BINDER, Karoline, 81241 München (DE); MAYER, Rebecca, 86899 Landsberg am Lech (DE); SZALAY, Gabor, 35398 Gießen (DE)
(74) Representative: Lohr, Jöstingmeier & Partner

(57) **Abstract**

A surgical alignment device is disclosed herein, which can include a first bar and second bar, configured to be parallel displaced by the means of a displacement screw. A first and a second wire holder, configured to receive a wire, may be configured displaceable within the first bar and the second bar.

## Description

### Background

The disclosure relates to surgical devices, and more particularly to a device for aligning bones during open surgery.

### Summary

Disclosed herein are surgical devices that can align bones during open surgery. Embodiments may be directed to an alignment device which may include a bone fragment manipulator. Any bone fragment manipulator may include a bone penetrating member.

In an embodiment, an alignment device may include at least two bars, a displacement guide and a parallel displacement means (for example, a screw), perpendicular to said bars. A first bar and a second bar may be configured for parallel displacement of the first bar relative to the second bar. Each bar may independently include a straight portion and a curved portion. An alignment device can further include at least two wire holders. For example, an alignment device can include a first wire holder configured for holding a first wire and a second wire holder configured for holding a second wire. A wire may be a K-wire. The parallel displacement screw may include a displacement screw handle, which may have a knurled surface.

In an embodiment, the parallel displacement screw may include two oppositely threaded portions, which may interact with threaded holes corresponding to the respective thread within the first bar and the second bar. In another embodiment the parallel displacement screw may have a single thread, with the screw penetrating one bar in an unthreaded hole and interacting with an inner thread in the other bar. The displacement screw may be a hexagon socket screw. The displacement screw handle may be located at the first bar or at the second bar.

By rotating the parallel displacement screw, a compression force may be applied to the first bar and/or the second bar, and whereby the bars may be displaced.

In an embodiment, the displacement guide may be an unthreaded rod or bolt, which may be fixed in the first bar on one end and be slidably mounted within a corresponding bore within the second bar, or vice versa. The displacement guide may closely fit in the bore and may have a gap of 0.1 mm - 0.3 mm to avoid an unsteady movement and ensure an accurate parallel displacement. In a different embodiment, there may be two displacement guides with the same characteristics as in the preceding embodiment. In another embodiment, the displacement guide may be a scissor type guide.

In an embodiment, the first wire holder may include a first wire support, a first clamp screw, and a first adjustment handle. The second wire holder may include a second wire support, a second clamp screw, and a second adjustment handle. The first wire holder and the second wire holder may be identical. The first adjustment handle and the second adjustment handle may independently have a knurled surface. The first wire holder may be configured for a longitudinal displacement within the first bar. The second wire holder may be configured for a longitudinal displacement within the second bar. The displacement may be a longitudinal sliding motion of the respective wire holders within the bars. In an embodiment, the first wire holder may be placed in a first slot within the first bar, and the second wire holder may be placed in a second slot within the second bar. The slots may be elongated, arc shaped, and located within the curved portions of the respective bars. Any slot may be limited by a first stop and second stop. In further embodiments, at least one wire holder may be configured for displacement, while the other wire holder may be in a fixed position. In an embodiment, the first wire holder may be held at the first bar, while the second wire holder may be configured to be displaceable. In another embodiment, the first wire holder may be configured to be displaceable, while the second wire holder may be held at the second bar.

The wire holders may be configured for tilting about an axis of rotation located offset and below the alignment device, by displacing the wire holders in the respective arc shaped slots. The first wire holder and the second wire holder may be tilted independently to each other. The axis of rotation of the first wire holder and the first wire may be located in a first bone if the first bar is positioned above the first bone. The axis of rotation of the second wire holder and the second wire may be located in a second bone if the second bar is positioned above the second bone. The axes of rotation of said wire holders may be essentially the same. The first wire and second wire, which may be K-wires, may be received by a bore within the first wire support and the second wire support.

The first clamp screw and the second clamp screw may have a male thread, whereas the first adjustment handle and the second adjustment handle may be a nut. By rotating the first adjustment handle and the second adjustment handle the threads may be interacting and said adjustment handles may be tightened or untightened. As a consequence, the first wire holder, including the first wire, and the second wire holder, including the second wire, may be locked in place or loosened independently to each other, depending on the direction of the rotation. In addition to that, by tightening said adjustment handles, the first wire may be pressed against the inner face of the first bar and the second wire may be pressed against the inner face of the second bar. As a result, the first wire and the second wire may be locked in place.

In an embodiment, the first bar and the second bar may have length of about 4.0 cm - 6.0 cm, a width of 0.25 cm - 0.75 cm and height of 0.75 cm - 1.25 cm. The supports may have a width of about 0.4 cm, may be 0.6 cm high and include a wire receiving bore, with a diameter of preferably about 0.15 cm - 0.25 cm.

The wires, which may be K-wires, may have a diameter of about 0.10 cm - 0.22 cm.

An embodiment relates to a method of aligning a first bone and a second bone. The first wire and the second wire, which may be K-wires, may be engaged with a first bone and a second bone. In a subsequent step, the alignment device is placed over the wires, with the fist wire being received by the first wire support and the second wire being received by the second wire support. The alignment device may be positioned onto the bones. In an embodiment, the curved portion of the first bar is positioned at or above the first bone and the curved portion of the second bar is positioned at or above the second bone.

The bones may be aligned by tilting the first wire and the second wire about an axis of rotation within the respective bones. The tilt may be accomplished by displacing or shifting the first wire holder and second wire holder within the first slot and the second slot. The wire holders may be displaced by moving the adjustment grips manually or by tilting the wires in a way that the wire holders may be displaced along with the wires, with the respective notches guiding the movement. The wires and the wire holders may be tilted independently about an axis of rotation offset to the device. The tilt of the first wire may be locked in place by tightening the first adjustment handle and the tilt of the second wire may be locked in place by tightening the second adjustment handle. The adjustment handles may be tightened or untightened by the rotation of the adjustment handles. A parallel compression may be applied by tightening the displacement screw, which is interacting with the first bar and the second bar.

The alignment device provides convenient handling and easy adjustment of the bones' positions. A secure hold without divergence of the alignment device is accomplished by curving a portion of the bars, thus the alignment device may be accurately fitted onto the bones by the curved portions. The comparatively light weight and the simple mechanics of the alignment device benefit the handling as well. A precise and convenient alignment of the bones is furthermore accomplished by providing means of tilting the wires independently about a rotation axis located offset to the device within the bone. An offset rotation axis causes less divergence and reduces the risk of a deficient alignment. The tilt of the wires, and therefore the position of the bones, can be locked in place independently. Due to this feature, the risk of a failed alignment, caused by holding the wires in place manually, is reduced. In addition, a compression force can be easily applied to the wires engaged with the bone by the displacement screw. The rotation of the screw provides a sensitive adjustment of the parallel displacement.

When aligning and reducing the scaphoid and lunate during open surgery, an alignment without divergence is required. A failed scaphoid and lunate position can cause arthrosis, which can ultimately lead to a Scapholunate Advanced Collapse. With an accurate alignment and the right amount of compression, a precise operation is possible.

The alignment device comprises few components and is further characterized by its small size and light weight. As a consequence, little material is needed for the production of the alignment device, therefore the cost of material and the manufacturing costs may be comparatively low. In addition to that, the manufacturing process of the individual parts of the alignment device is simple and the assembly may be convenient. Therefore, mass production is possible. To safe costs, the parts of the alignment device may be sold as a self-assembly kit, due to its simple mechanical construction.

### Detailed Description

In the following, embodiments will be described by way of example.
Figure 1 shows an embodiment of the alignment device.
Figure 2 shows a top view of an embodiment of the alignment device.
Figure 3 shows a cross-sectional view of an embodiment of the alignment device.
Figure 4 shows a further cross-sectional view of an embodiment of the alignment device.
Figure 5 shows a cross-sectional view of an embodiment of the alignment device with a tilted wire.
Figure 6 shows an embodiment of the alignment device interacting with bones.
Figure 7 shows an embodiment of the alignment device about a rotation axis.

The embodiments may be directed to an alignment device which may include a bone fragment manipulator. A bone fragment manipulator may include a bone penetrating member.

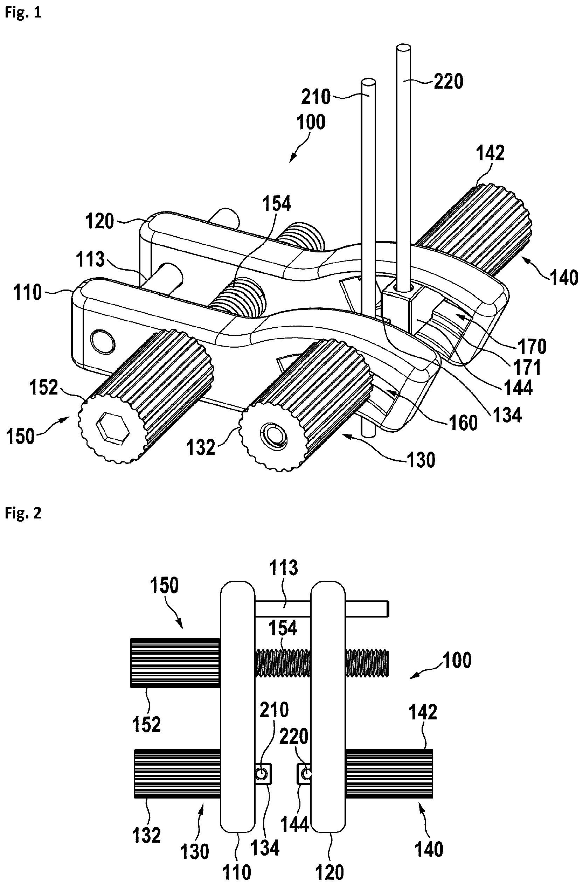

In figure 1 an embodiment of an alignment device (100) is shown. The alignment device may include a first bar (110) and a second bar (120) held essentially parallel thereto. The first bar (110) and the second bar (120) may be connected by a displacement guide (113) and a parallel displacement screw (150). By the rotation of the parallel displacement screw, the distance of the first bar (110) and the second bar (120) may be increased or decreased. The displacement guide (113) provides a parallel displacement of the first bar (110) relative to the second bar (120). In this embodiment, a first wire holder (130) may be located in a first slot (160) within the first bar (110) and a second wire holder (140) may be located in a second slot (170) within the second bar (120). The first slot and the second slot may be limited by a first stop (171) and a second stop (172). The first wire holder (130) may include a first adjustment handle (132), a first wire support (134) and a first clamp screw (135). The second wire holder may include a second adjustment handle (142), a second wire support (144) and a second clamp screw (145). The first adjustment handle (132) and the second adjustment handle (142) may have a knurled surface. The first wire holder (130) may be configured displaceable within the first slot (160) and the second wire holder (140) may be configured displaceable within the second slot (170). In an embodiment, the first slot (160) and the second slot (170) may be elongated and arc shaped. The first wire (210) may be held in a bore within the first wire support (134) and second wire (220) may be held in a bore within the second wire support (144). The wires (210, 220) may be K-wires. The elongated, arc shaped slots (160, 170) may guide the wire holders (130, 140), including the wires (210,210), to a tilting movement about an offset rotation axis outside the bars (110, 120). In this embodiment, the parallel displacement screw (150) may include a displacement handle (152), which may have a knurled surface, and a displacement thread (154). The displacement handle (152) may be located at the first bar. The parallel displacement screw (150), which may be a hexagon socket screw, may include a single thread passing through the first bar in an unthreaded hole and being received by an inner thread within the second bar (220). In another embodiment the displacement thread (154) may include two oppositely threaded portions, which are received by threaded holes corresponding to the respective thread within the first bar (110) and the second bar (120). The displacement handle (152) may be located at the first bar (110) or at the second bar (120). In this embodiment, the displacement guide (113) may be an unthreaded rod fixed in the first bar (110) and slidable mounted within a corresponding bore within the second bar (120). In a different embodiment, there may be at least one displacement guide (113), equivalent to the previous displacement guide (113). In another embodiment, the displacement guide (113) may include a second screw parallel to the parallel displacement screw (150). The first bar (110) and second bar (120) each include a straight portion and a curved portion. The curved portion may be arched to the same degree as the respective slots (160, 170).

In figure 2 a top view of an embodiment of an alignment device (100) is shown. The first bar (110) and the second bar (120) are parallel displaced. The displacement guide (113) and parallel displacement screw (150) may be positioned perpendicular to the first bar (110) and second bar (120). In an embodiment, the displacement handle (152), which may have a knurled surface, may be positioned at the first bar (110), with the displacement thread (154) interacting with at least one of the bars (110, 120). The first wire holder (130) may be moveable within the first bar (110), the second wire holder (140) maybe moveable within the second bar (120). The first wire (210) may be received by the first wire support (134) and the second wire (220) may be received by the second wire support (144). The first wire (210) and the second wire (220) may be K-wires. The first adjustment handle (132) may be located at the outer face of the first bar (110) and the second adjustment handle (132) may be located at the outer face of the second bar (120). In an embodiment, the first adjustment handle (132) and the second adjustment handle (142) may have a knurled surface. The first wire support (134) and the first wire (210) as well as the second wire support (144) and the second wire (220) may be positioned in the recess between the fist bar (110) and the second bar (120).

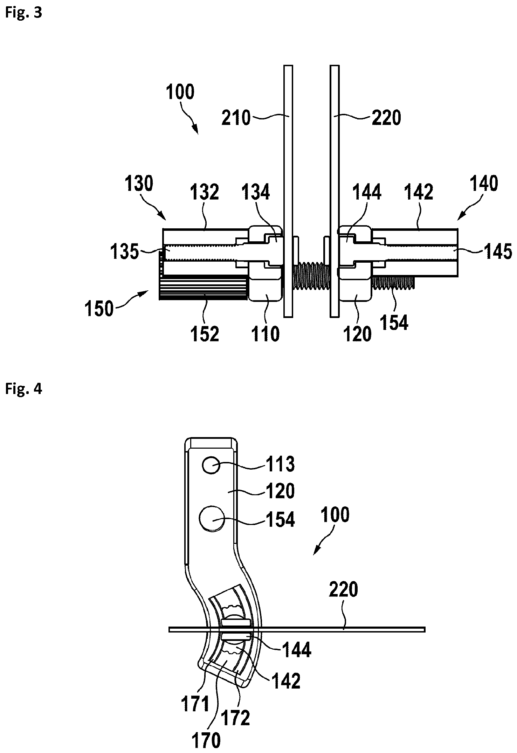

In figure 3 a cross-sectional view of the first wire holder (130), the second wire holder (140), the first bar (110) and the second bar (120), with a viewing direction towards the parallel displacement screw (150), of an embodiment of the alignment device (100) is shown. The cross section shows the first clamp screw (135) and the second clamp screw (145) as well as the first adjustment handle's (132) and second adjustment handle's (142) inner threads. The cross-sectional view of the first wire holder (130) shows the first clamp screw (135) and the first wire support (134) as one unit. The cross sectional view of the second wire holder (140) shows the second clamp screw (145) and the second wire support (144) as one unit. The first clamp screw (135) is interacting with the inner thread of the first adjustment handle (132) and the second clamp screw (145) is interacting with the inner thread of the second adjustment handle (142). The first wire holder (130) and the second wire holder (140), including the first wire (210) and the second wire (220) may be locked in place independently by tightening the respective adjustment handle (132, 142). The first wire holder and the second wire holder, including the first wire (210) and the second wire (220), may be loosed by untightening the respective adjustment handles (132, 142). The adjustment handles (132, 142) may be tightened or untightened by rotation. In an embodiment, the displacement handle (152) is located on the first bar's (110) outer surface. The displacement thread (154) is interacting with an inner thread in at least one of the bars (110,120).

In figure 4 the inner face of the second bar (120) and the second adjustment handle (142) with a cross sectional view of the displacement guide (113), the displacement thread (154) and the second wire support (144), of an embodiment of the alignment device (100) is shown. The second wire support (144) is holding the second wire (220). The second bar (120) includes a second slot (170) with a first stop (171) and a second stop (172). Said slot (170) may have an arched shape, with the respective portion of the bar (120) adapting to said contour. The second wire holder (140) may slide along the slot (170) in both directions, thus the second wire (220) may be tilted about an axis of rotation offset to the device.

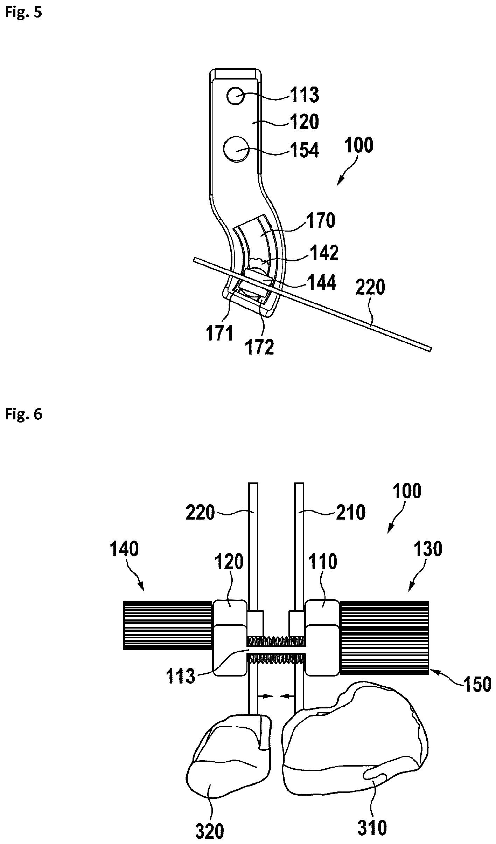

Figure 5 matches figure 4 with the exception of the second adjustment grip (142) and second wire support (144), including the second wire (220), being displaced to one end of the second notch (170) and therefore tilted to the full extent in one direction.

In figure 6 an embodiment of the alignment device (100) is shown. The first wire (210) is engaged with the first bone (310) and the second wire (220) is engaged with the second bone (320). The first bar (110) and the second bar (120) may be parallel displaced by the parallel displacement screw (150) and guided by the displacement guide (113). The rotation of the parallel displacement screw (150) applies a compression force to the first bar (110) and the second bar (120). The first wire holder (130) and the second wire holder (140) may tilt the first wire (210) and second wire (220), which may be K-wires, and align the first bone (310) and the second bone (320).

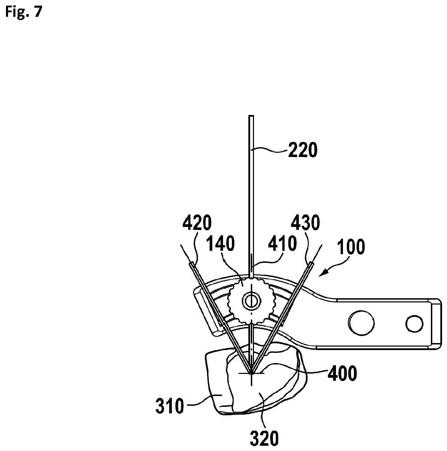

In figure 7 a view towards the outer face of an embodiment of the alignment device (100) is shown. In this embodiment, the second wire (220) is engaged with the second bone (320). The wire (220) is located in a center position (410) with no tilt. In this embodiment, the wire (220) may be tilted about an axis of rotation (400) located within the second bone (320). The wire (220), which may be a K-wire, may be tilted to a first maximum tilt (420) and a second maximum tilt (430). Said maxima representing the radius of the possible tilt.

### List of reference numerals

- 100: alignment device
- 110: first bar
- 113: displacement guide
- 120: second bar
- 130: first wire holder
- 132: first adjustment handle
- 134: first wire support
- 135: first clamp screw
- 140: second wire holder
- 142: second adjustment handle
- 144: second wire support
- 145: second clamp screw
- 150: parallel displacement screw
- 152: displacement handle
- 154: displacement thread
- 160: first slot
- 170: second slot
- 171: fist stop
- 172: second stop
- 210: first wire
- 220: second wire
- 310: first bone
- 320: second bone
- 400: axis of rotation
- 410: center axis
- 420: first maximum tilt
- 430: second maximum tilt

## Claims

1. An alignment device (100), comprising:
a first bar (110) and a second bar (120), the first bar (110) and the second bar (120) being connected by a parallel displacement screw (150) and configured for parallel displacement by rotation of the parallel displacement screw (150),
at least one displacement guide (113), configured for guiding the parallel displacement of the first bar (110) relative to the second bar (120),
a first wire holder (130) displaceable within a first slot (160) of the first bar (110),
a second wire holder (140) held at the second bar (120),
said wire holders (130, 140) including wire supports (134, 144), said wire supports being (134, 144) configured for receiving a wire (210, 220);

2. The alignment device (100) according to claim 1,
**characterized in, that**
the first bar (110) includes a first curved section holding the first slot (160).

3. The alignment device (100) according to any of the previous claims,
**characterized in, that**
the first slot (160) is arc shaped and the first wire holder (130) is guided within the first slot (160) such that displacement of the first wire holder (130) within the first slot (160) results in a tilting movement of the first wire holder (130) about an axis (400) outside of the first bar (110).

4. The alignment device (100) according to claim 1 or 2,
**characterized in, that**
the second wire holder (140) is displaceable within a second slot (170) of the second bar (120).

5. The alignment device (100) according to claim 4,
**characterized in, that**
the second bar (120) includes a second curved section holding the second slot (170).

6. The alignment device (100) according to claim 4 or 5,
**characterized in, that**
the second slot (170) is arc shaped and the second wire holder (140) is guided within the second slot (170) such that displacement of the second wire holder (140) within the second slot (170) results in a tilting movement of the second wire holder (140) about an axis (400) outside of the second bar.

7. The alignment device (100) according to any of the previous claims,
**characterized in, that**
at least one wire support (134,144) is configured to fix a wire (210,220) held by the wire support (134,144) in its position relative to a bar (110,120)

8. The alignment device (100) according to any of the previous claims,
**characterized in, that**
the displacement screw (150) is interacting with a thread (154) in the second bar (120) and
the displacement screw (150) is either interacting with an internal thread in the first bar (110) or is passing through an unthreaded hole in the first bar (110).

9. The alignment device (100) according to any of the previous claims,
**characterized in, that**
the displacement guide (113) is a rod fixed to the first bar (110) or the second bar (120), guiding the other bar within a bore.

10. The alignment device (100) according to any of the previous claims,
**characterized in, that**
at least one of the wires (210, 220) is a K-wire.

11. The alignment device (100) according to any of the previous claims,
**characterized in, that**
at least one wire holder (130,140) is configured to hold at least one of the wires (210, 220) in its position by pressing the wire (210, 220) against a side of a bar (110,120).

12. The alignment device (100) according to any of the previous claims,
**characterized in, that**
at least one adjustment handle (132, 142) and displacement handle (152) has a knurled surface.
